# EUROPEAN PATENT SPECIFICATION

(11) **EP 2 732 841 B1**
(45) Date of publication and mention of the grant of the patent: **01.03.2017**
(21) Application number: 12192582.0
(22) Date of filing: 14.11.2012
(51) Int. Cl.: A61M 16/20, G01F 1/22, F17C 13/00, F17D 1/02, G05D 16/00, G05D 7/00, G01F 15/18, A61M 16/00, A61M 16/12

(54) **Variable area flowmeter for dosing medical gases**
Variabler Bereichsdurchflussmesser zur Dosierung medizinischer Gase
Débitmètre à section variable pour le dosage des gaz médicaux

(43) Date of publication of application: 21.05.2014
(73) Proprietor: Flow Meter S.p.a., 24040 Levate (Bergamo) (IT)
(72) Inventor: Paratico, Roberto, 24040 Levate (Bergamo) (IT)
(74) Representative: Botti, Mario

(56) References cited:
- EP-A1- 1 462 140
- EP-A2- 1 562 026
- FR-A1- 2 645 027
- FR-A1- 2 763 382
- FR-A1- 2 800 285
- GB-A- 191 012 857
- US-A- 3 232 107
- US-A- 3 586 045
- US-A- 3 949 966
- US-A- 4 114 607
- US-A- 4 241 896
- US-A- 4 245 513
- US-A- 6 116 242

## Description

### Field of application

The present invention relates to an instantaneous variable area flowrate measuring device - otherwise known as flowmeter - designed to dose medical gases

### Prior Art

In various applications in different technological fields it is required to perform the regulation and measurement of the flowrate of a gas or a generic fluid flowing in circuits or pipes.

In particular, in the medical sector, regulators and flowrate measuring devices for dosing the supply of pressurised gas intended for therapeutic applications, such as oxygen therapy, are used. These regulators, called flowmeters in the sector since they allow instantaneous reading of the flowrate of the fluid supplied, may be applied to the outlet of a distribution network or, if a suitable highpressure reducer is arranged in between, they may be applied to a pressurised vessel containing medical gas.

These devices, which have a simple structure and small volume, incorporate internally a variable area flowrate measuring device (the actual flowmeter) and a regulating valve which allows variation of the flow supplied, with the possibility of reading in real time the selected flowrate from a graduated column of the flowrate measuring device.

The devices of the type described above, although substantially satisfying the requirements of the sector, nevertheless have a number of drawbacks which hitherto have not been overcome.

A first major drawback relates to calibration of the flowmeter. Each model is calibrated with reference to a given operating pressure. Although the flowmeters are normally associated with medical gas distribution networks which have output pressures defined within specific ranges determined by different reference standards, these pressures may nevertheless be subject to variations also of a substantial nature due, for example, to the number of users connected to the system at the same time. Moreover, the standards of different countries may envisage pressure values which also differ significantly from each other.

Another drawback of the devices according to the prior art also relates to the corresponding difficulty of reading the values from the graduated scale of the flowrate measuring device; it is not always possible to increase the size of this component since small dimensions are an often vital requisite in medical applications.

Yet another drawback relates to the use of these devices owing to the external accessibility of the knob for driving the regulating valve. It happens, in fact, that during continuous use, this knob may be accidentally knocked by medical or other staff.

Another drawback consists in the specific nature of the threaded connection provided at the outlet. In the medical sector, in fact, a single set of regulations defining a single globally recognised standard for threaded connections does not exist. Instead, there exist a series of national regulations which propose standards valid for a particular country, together with other type-approval certifications which categorize similar appliances.

Documents EP 1 462 140 A1; US 6,116,242 A; EP 1 562 026 A2; FR 2 800 285 A1; US 3, 586 045 A; and FR 2 645 027 A1 disclose a number prior art devices provided for controlling the flow rate of a medical gas in several applications.

The technical problem forming the basis of the present invention is therefore that of devising a flowmeter which has structural and functional characteristics such as to overcome the drawbacks mentioned above in connection with the prior art and which solves in particular the problems of the measuring precision when the pressure fluctuates.

### Summary of the invention

The aforementioned technical problem is solved by a flowmeter for dosing medical gases, comprising a main body which has, defined therein, at least one flow supply path extending between an inlet and an outlet; said flowmeter comprising at least one flow regulation stage which allows continuous variation of the flowrate of the medical gas supplied at the outlet and at least one flowrate measuring device arranged downstream of said flow regulation stage; said flowmeter also comprising a pressure stabilization stage arranged so as to intercept said flow supply path upstream of the flow regulation stage.

A person skilled in the art will appreciate immediately how, owing to the presence of the stabilization stage upstream of the device, it is possible to avoid pressure fluctuations which negatively influence the measuring accuracy of the device and to adapt to the different supply pressure values defined by the single national standards.

The flow regulator may comprise a regulating valve driven by a regulation knob (for example a needle valve) which is accessible externally with respect to said main body.

In this case, in order to avoid problems of accidental movement of the regulation knob, it may be movable linearly between a regulating configuration, where it is rotationally movable with respect to the main body and drives the regulating valve, and a locking configuration, where it is rotationally locked with respect to the main body.

The flowrate measuring device is preferably of the variable area type and comprises a reading column which communicates with the flow supply path and slidably houses internally a float - typically a ball - the position of which indicates the flowrate.

The reading column, which has a graduated scale, may be contained inside an outer casing which is at least partly transparent and defines a magnifying lens intended to facilitate reading of said graduated scale.

The outer casing may in this case have a substantially oval cross-section in order to facilitate formation of the magnifying lens incorporated in the glass.

In particular, it may comprise: a piston with a disc facing said internal chamber and a stem sealingly sliding inside a guide bush communicating with the inlet; and resilient means associated with the piston and designed to oppose the fluid pressure inside the internal chamber; the stem of said piston having an inner channel which connects the guide bush to the internal chamber, the access to which is interrupted when said stem reaches an end-of-travel position inside the guide bush.

The main body is composed of a plurality of shells joined together, said guide bush being integrally formed on a first shell, the internal chamber being bounded by the inner walls of a second shell.

In particular, the reading column may be supported by a plate which is slidably inserted on top of said second shell.

The flowmeter may also comprise a stop flow stage which intercepts said flow supply path upstream of said pressure stabilization stage, said stop flow stage comprising a valve which can be operated from outside the main body and is designed to interrupt selectively the flow of medical gas.

The stop flow stage allows advantageously the supply to be interrupted without modifying the flowrate setting selected by means of the regulation knob which drives the needle valve.

The aforementioned valve may be a slide valve provided with a stem inserted inside a diametral hole of the main body, the opposite ends of said stem having two pushbuttons alternately projecting from an outer surface of the first shell.

The flowmeter comprises in general a threaded fitting for connection to a user appliance. This device may comprise advantageously means for fastening the threaded fitting to the main body, which can be released from outside the main body so as to allow removal of said threaded fitting.

This release facility allows easy replacement of the fitting depending on the connection requirements.

The threaded fitting may in particular have a first threaded connector and an opposite second threaded connector, said threaded fitting being able to be alternately positioned in two operating configurations where the first threaded connector or the second threaded connector respectively project towards the outside of the main body.

The two different connectors may thus comply with two different connection standards, ensuring an advantageous flexibility of use of the device.

Further characteristic features and advantages of the flowmeter according to the invention will emerge from the description, provided hereinbelow, of an example of embodiment, provided by way of a nonlimiting example, with reference to the accompanying drawings.

### Brief description of the drawings

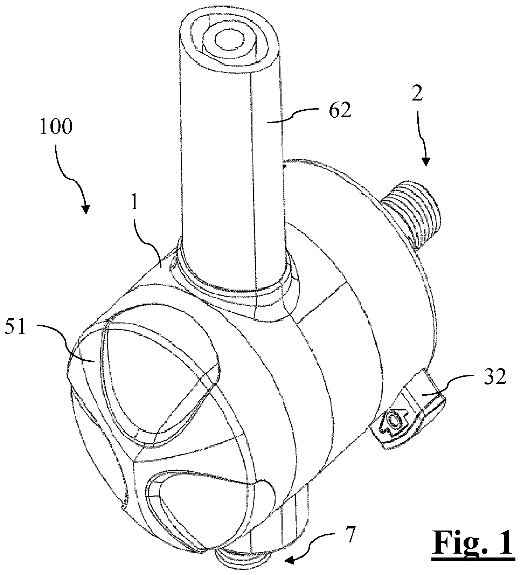

Figure 1 shows a perspective view of a flowmeter according to the present invention;
Figure 2 shows a side view of the flowmeter according to Figure 2, with the regulation knob in the locked configuration;
Figure 3 shows a view of the flowmeter according to Figure 1, with the regulation knob in the released configuration;
Figure 4 shows a side view, cross-sectioned along a middle plane, of a detail of the flowmeter according to Figure 1, with the regulation knob in the locked configuration;
Figure 5 shows a view, cross-sectioned along a middle plane, of a detail of the flowmeter according to Figure 1, with the regulation knob in the released configuration;
Figure 6 shows a front view of the flowmeter according to Figure 1, with the slide valve in the open configuration;
Figure 7 shows a front view of the flowmeter according to Figure 1, with the slide valve in the closed configuration;

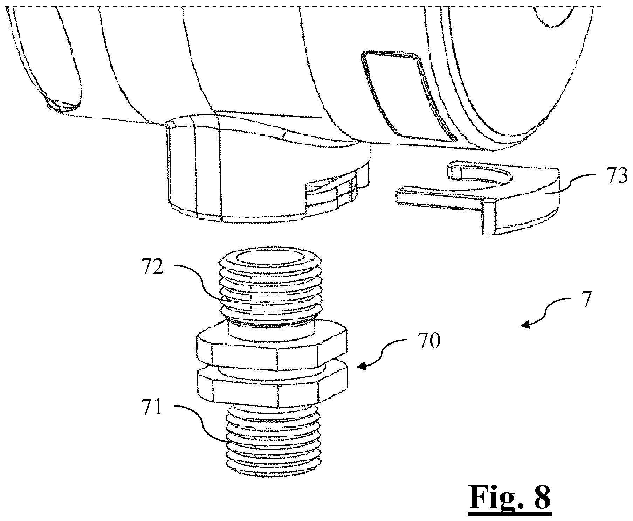

Figure 8 shows a perspective view of a detail of the flowmeter according to Figure 1;
Figure 9 shows an exploded perspective view of the flowmeter according to Figure 1.

### Detailed description

With reference to the attached figures, 100 denotes in general a variable area flowmeter, intended principally for dosing medical gases.

The flowmeter 100 comprises a main body 1, with a supporting and containment function, inside which at least one flow supply path extending between an inlet 2 - defined by a threaded connector of the known type - and an outlet 7 is defined.

The body 1 has a substantially cylindrical form coaxial with the threaded connector which defines the inlet 2; the outlet 7 instead has a radial orientation with respect to said main body 2.

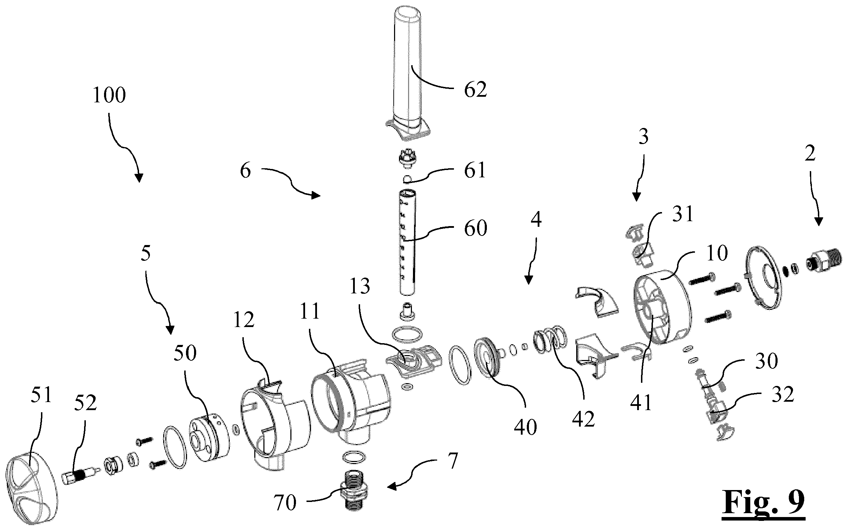

The main body 1 may be made of metallic material, for example anodized aluminium or chrome-plated brass, or by means of injection-moulding of polymer material, for example polycarbonate, or also may be made partly of metallic material and partly of plastic material. In fact, as can be seen in Figure 9, the main body 1 is composed of different portions obtained separately and optionally made of different materials.

Along the flow supply path there are arranged in series, in an upstream to downstream direction: a stop flow stage 3, a pressure stabilization stage 4, a flow regulation stage 5 and a flowrate measuring device 6.

The stop flow stage 3 is formed inside a first shell 10 of the main body 1 made as a single piece by means of moulding.

Said first shell 10, with an essentially cylindrical outer form, is passed through transversely by a slide valve 30 which intercepts the flow supply path.

The slide valve 30 has a stem inserted inside a diametral hole of the first shell 10. This stem is movable between a closing position, in which it blocks the flow supply path, and an open position, in which said flow supply path is not blocked.

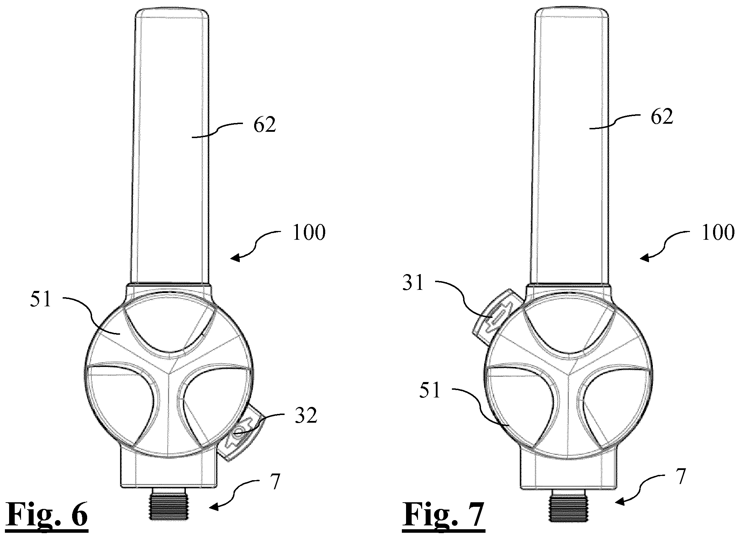

Two oppositely arranged pushbuttons 31, 32 are integral with the ends of the stem and project alternately from the cylindrical surface of the main body 1 depending on whether the slide valve 30 is in the open configuration, as can be seen in the accompanying Figure 6, or in the closed configuration, as can be seen in the accompanying Figure 7.

It should be noted that the diametral hole which receives the stem has an offset axis, preferably inclined at 45°, with respect to the vertical middle plane along which the outlet 7 lies. This orientation of the slide valve 30 allows the overall dimensions to be advantageously reduced. The main body also comprises a second shell 11, which also has a substantially cylindrical shape and is made by means of injection-moulding and which is fastened to the first shell 10 by means of three locking screws.

The contact ends of the two shells are shaped so as to fit together, defining externally the cylinder of the main body 1; an internal volume which houses the pressure stabilization stage 4 is instead formed between said shells.

This stage comprises a piston 40, with a stem which is sealingly inserted inside a guide bush 41 integrally formed with the first shell 10 and which communicates with the inlet 1 opposite thereto. The disc of the piston 40 is instead sealingly slidable inside a cylindrical seat which is formed internally in the second shell 11 and which communicates with the remainder of the flow supply path.

An internal chamber is thus created downstream of the disc; a compression spring 42 which opposes the fluid present inside said internal chamber is arranged between disc and first shell 10.

The stem of the piston 40 has an inner channel which connects the guide bush 41 to the internal chamber and therefore ensures the continuity of the flow supply path. The inner channel and the guide bush 41 are however formed so that, when the piston 40 is pushed to the end of its travel against the bottom of the bush, access to the inner channel is interrupted. An interruption of the flow is thus obtained whenever the pressure inside the internal chamber exceeds a threshold value determined by the rigidity of the compression spring 42, thus achieving the desired stabilization of the pressure.

The flow regulation stage 5, which is arranged immediately downstream of the aforementioned pressure stabilization stage 4, comprises a needle valve 50 for regulating the flowrate.

Said valve, the internal details of which are not visible in the accompanying figures, is formed inside a cylindrical body which is sealingly engaged inside the second shell 11, closing it at the front.

The movable closing member is arranged at the end of an actuating stem 52 fastened by means of a threaded connector to the aforementioned cylindrical body. Rotation of an end head of the actuating stem 52 thus produces a linear movement of the closing member which varies the flowrate of the medical gas supplied at the outlet 7.

The aforementioned end head is rotationally coupled, by means of a form-fit connection, to a regulation knob 51 outside of the main body 1, so that the regulation knob 51 drives the needle valve 50. The regulation knob 51 is, however, free to move along the axis of the actuating stem 52.

The regulation knob 51 has, in particular, a cup-like form and has a raised inner rim 53. The regulation knob 51 is fitted on top of a cylindrical collar of the second shell 11, and the inner rim 53 thus cooperates with projections 54, 55 formed on the outer surface of said cylindrical collar.

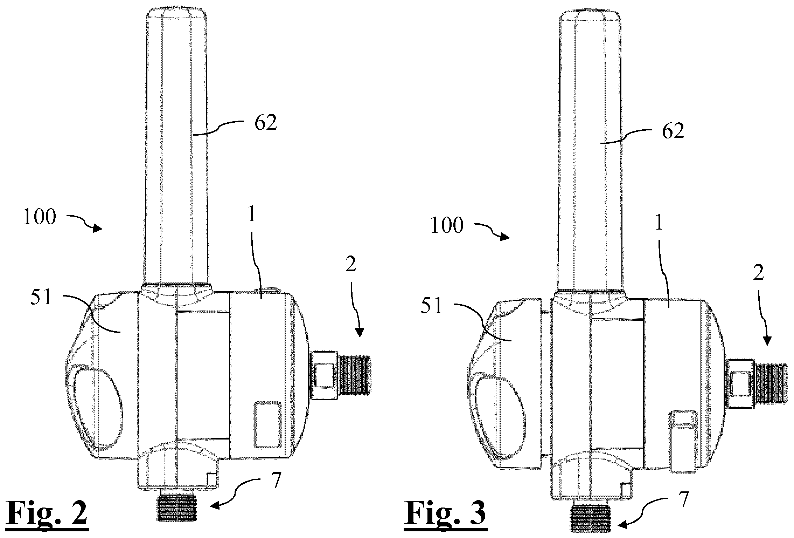

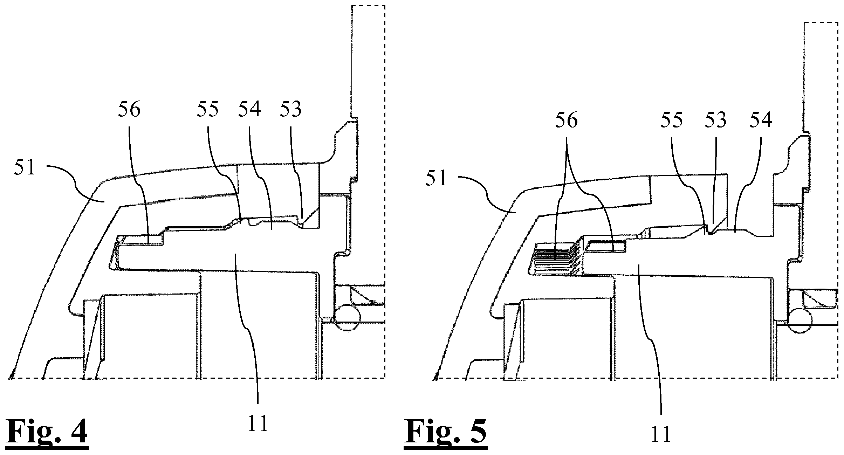

A first projection 54 connected by means of ramps to the surface of the cylindrical collar and with a height less than that of the second projection 55 is designed to keep the regulation knob 51 in a locked configuration, shown in Figures 3 and 5, where the cylindrical collar is completely inserted inside the regulation knob 51.

It should be noted that the outer rim of the cylindrical collar, as well as the base of the side wall of the regulation knob 51, are provided with sets of straight-teeth toothing 56 which engage with each other when the regulation knob 51 is in said locked position. Rotation of the knob with respect to the main body 1 is therefore not possible.

By pulling the regulation knob 51 outwards, it is however possible to move the inner rim 53 beyond the first projection 54, until it is locked against the second projection 55 in a released configuration of the regulation knob 51 shown in Figures 4 and 6.

In this released configuration, the teeth 56 are not engaged with each other and it is thus possible to operate the regulation knob 51 in order to dose the flowrate of the medical gas supplied.

The main body 1 comprises a third shell 12, with a substantially annular shape, associated with the second shell 11 and arranged so as to cover partly the regulation knob 51.

The flowrate measuring device 6, arranged downstream of the flow regulation stage 5 described above, is of the variable area type.

It comprises a cylindrical and transparent reading column 60 which communicates with the flow supply path and slidably houses internally a float 61 - in particular a ball - the position of which indicates the flowrate of the medical gas supplied.

A graduated scale, which allows the flowrate value to be determined based on the position of the ball, is present on the reading column.

An outer casing 62 with an oval cross-section covers externally the reading column 60. Said outer casing, which is made of glass or other transparent material, incorporates a magnifying lens intended to facilitate reading of the graduated scale of the underlying reading column 60.

The reading column 60 and the outer casing 62, which protrude vertically above the main body 1, are both supported by a plate 13 which slidably engages in an upper impression formed in the second shell 11.

The outlet 7 of the device opens out at the end of a sleeve projecting radially from the main body 1. A seat is formed inside said sleeve and has the function of housing a threaded fitting 70 of said outlet 7.

The threaded fitting 70 has a first threaded connector 71 at one of its ends and a second threaded connector 72 at the opposite end. The two threaded connectors have different diameter sizes and/or thread parameters.

A non-threaded central part provided with a circumferential recess is present between the two threaded connectors 71, 72.

In the operating configuration, one of the two threaded connectors 71, 72, as required, is inserted inside the seat defined above; the other connector instead projects beyond the projecting sleeve so as to allow connector of a sanitary appliance. Thus, by reversing the orientation of the sleeve, it is possible to obtain two different operating configurations, depending on whether the first or second connector is positioned externally.

Fastening means are provided in order to allow the sleeve to be locked in either one of its operating configurations.

These fastening means comprise a pin 73 which is inserted inside a slot formed in the projecting sleeve and engages with the circumferential recess of the threaded fitting 70.

Obviously, a person skilled in the art, in order to satisfy any specific requirements which arise, may make numerous modifications and variations to the flowmeter described above, all of which being moreover contained within the scope of protection of the invention, as defined by the following claims.

## Claims

1. Flowmeter (100) for dosing medical gases, comprising a main body (1) which has, defined therein, at least one flow supply path extending between an inlet (2) and an outlet (7); said flowmeter (100) also comprising at least one flow regulation stage (5) which allows continuous variation of the flowrate of the medical gas supplied at the outlet (7) and at least one flowrate measuring device (6) of the variable area type comprising a reading column (60) which communicates with the flow supply path and slidably houses internally a float (61), the position of which indicates the flowrate; said flowrate measuring device (6) being arranged downstream of said flow regulation stage (5); wherein it also comprises a pressure stabilization stage (4) arranged so as to intercept said flow supply path upstream of the flow regulation stage (5), wherein said pressure stabilization stage (4) is designed to interrupt the flow when the pressure of the medical gas inside an internal chamber upstream of the flow regulation stage (5) exceeds a threshold value, **characterized in that** said pressure stabilization stage (4) comprises: a piston (40) with a disc facing said internal chamber and a stem sealingly sliding inside a guide bush (41) communicating with the inlet (2); and resilient means associated with the piston (40) and designed to oppose the fluid pressure inside the internal chamber; the stem of said piston (40) having an inner channel which connects the guide bush (41) to the internal chamber, the access to which is interrupted when said stem reaches an end-of-travel position inside the guide bush (41), wherein said main body (1) is composed of a plurality of shells joined together, said guide bush (41) being integrally formed on a first shell (10), the internal chamber being bounded by the inner walls of a second shell (11); wherein said reading column (60) is supported by a plate (13) which is slidably inserted on top of said second shell (11).

2. Flowmeter (100) according to Claim 1, wherein said reading column (60) has a graduated scale, said reading column (60) being contained inside an outer casing (62) which is at least partly transparent and defines a magnifying lens intended to facilitate reading of said graduated scale.

3. Flowmeter (100) according to Claim 2, wherein said outer casing (62) has a substantially oval cross-section.

## Patentansprüche

1. Durchflussmesser (100) zur Dosierung medizinischer Gase, umfassend einen Hauptkörper (1), welcher, darin definiert, mindestens einen Strömungszufuhrweg aufweist, der sich zwischen einem Einlass (2) und einem Auslass (7) erstreckt; wobei der Durchflussmesser (100) auch mindestens eine Durchflussregelungsstufe (5) umfasst, die eine kontinuierliche Variation der Durchflussrate des medizinischen Gases ermöglicht, das am Auslass (7) bereitgestellt wird, und mindestens eine Durchflussratenmessvorrichtung (6) vom Schwebekörper-Typ, umfassend eine Ablesesäule (60), die mit dem Durchflusszufuhrweg kommuniziert und innen gleitbar einen Schwebekörper (61) aufnimmt, dessen Position die Durchflussrate anzeigt; wobei die Durchflussratenmessvorrichtung (6) stromabwärts von der Durchflussregelungsstufe (5) angeordnet ist; wobei dieser auch eine Druckstabilisierungsstufe (4) umfasst, die derart angeordnet ist, dass sie den Durchflusszufuhrweg stromaufwärts von der Durchflussregelungsstufe (5) abfängt, wobei die Druckstabilisierungsstufe (4) ausgelegt ist, den Durchfluss zu unterbrechen, wenn der Druck des medizinischen Gases innerhalb einer inneren Kammer stromaufwärts von der Durchflussregelungsstufe (5) einen Schwellenwert überschreitet, **dadurch gekennzeichnet, dass** die Druckstabilisierungsstufe (4) umfasst: einen Kolben (40) mit einer Scheibe, die der inneren Kammer zugewandt ist, und einem Stempel, der abdichtend in einer Führungshülse (41) gleitet, die mit dem Einlass (2) kommuniziert; und elastische Mittel, die dem Kolben (40) zugeordnet und ausgelegt sind, dem Fluiddruck innerhalb der inneren Kammer entgegenzuwirken; wobei der Stempel des Kolbens (40) einen inneren Kanal aufweist, der die Führungshülse (41) mit der inneren Kammer verbindet, zu welcher der Zugang unterbrochen wird, wenn der Stempel eine Anschlagposition innerhalb der Führungshülse (41) erreicht, wobei der Hauptkörper (1) aus mehreren Schalen besteht, die miteinander verbunden sind, die Führungshülse (41) einstückig mit einer ersten Schale (10) gebildet ist, die innere Kammer durch die Innenwände einer zweiten Schale (11) begrenzt ist; wobei die Ablesesäule (60) von einer Platte (13) getragen ist, die der zweiten Schale (11) obenauf gleitbar eingesetzt ist.

2. Durchflussmesser (100) nach Anspruch 1, wobei die Ablesesäule (60) eine Mess-Skala aufweist, wobei die Ablesesäule (60) innerhalb eines Außengehäuses (62) enthalten ist, das mindestens teilweise transparent ist und eine Vergrößerungslinse definiert, die dazu bestimmt ist, das Ablesen der Mess-Skala zu erleichtern.

3. Durchflussmesser (100) nach Anspruch 2, wobei das Außengehäuse (62) einen im Wesentlichen ovalen Querschnitt aufweist.

## Revendications

1. Débitmètre (100) pour le dosage des gaz médicaux, comprenant un corps principal (1) qui a, défini intérieurement, au moins une voie d'alimentation en flux s'étendant entre une entrée (2) et une sortie (7); ledit débitmètre (100) comprenant aussi au moins un élément de régulation du flux (5), qui permet une variation continue du débit du gaz à usage médical fourni à la sortie (7) et au moins un dispositif de mesure du débit (6) du type à section variable, comprenant une colonne de lecture (60) qui communique avec la voie d'alimentation en flux et loge intérieurement un flotteur libre de coulisser (61), dont la position indique le débit ; ledit dispositif de mesure de débit (6) étant disposé en aval dudit élément de régulation (5) ; dans lequel
il comprend en outre un élément de stabilisation de la pression (4) agencé de manière à intercepter ladite voie d'alimentation en flux en amont de l'élément de régulation de débit (5), dans lequel ledit élément de stabilisation de pression (4) est conçu pour interrompre le flux lorsque la pression du gaz médical à l'intérieur d'une chambre interne en amont de l'élément de régulation de débit (5) dépasse une valeur de seuil, **caractérisé en ce que** ledit élément de stabilisation de la pression (4)
comprend : un piston (40) avec un disque faisant face à ladite chambre interne et une tige coulissant de manière étanche à l'intérieur d'un manchon de guidage (41) communiquant avec l'entrée (2) ; et des moyens élastiques associés au piston (40) et conçus pour s'opposer à la pression du fluide à l'intérieur de la chambre interne ; la tige dudit piston (40) ayant un canal interne qui relie le manchon de guidage (41) à la chambre interne, dont l'accès est interrompu lorsque la tige atteint une position de fin de course à l'intérieur du manchon de guidage (41), dans lequel ledit corps principal (1) est constitué d'une pluralité de coquilles reliées entre elles, ledit manchon de guidage (41) étant formé intégralement par une première coquille (10), la chambre interne étant délimitée par les parois intérieures d'une seconde coquille (11) ; dans lequel ladite colonne de lecture (60) est supportée par une plaque (13) qui est insérée de façon coulissante sur le dessus de ladite seconde coquille (11).

2. Débitmètre (100) selon la revendication 1, dans lequel ladite colonne de lecture (60) a une échelle graduée, ladite colonne de lecture (60) étant contenue à l'intérieur d'un boîtier extérieur (62) qui est au moins partiellement transparent et définit une lentille grossissante destinée à faciliter lecture de l'échelle graduée.

3. Débitmètre (100) selon la revendication 2, dans lequel ledit boîtier extérieur (62) a une section transversale sensiblement ovale.
